# FASCICULE DE BREVET EUROPEEN

(11) **EP 1 037 652 B1**
(45) Date de publication et mention de la délivrance du brevet: **16.10.2002**
(21) Numéro de dépôt: 98959996.4
(22) Date de dépôt: 15.12.1998
(51) Int. Cl.: A61K 38/08

(54) **COMPOSITIONS PHARMACEUTIQUES A BASE DE DALFOPRISTINE ET DE QUINUPRISTINE ET LEUR PREPARATION**
PHARMAZEUTISCHE ZUSAMMENSETZUNGEN AUS DALFOPRISTINE UND QUINUPRISTINE UND VERFAHREN ZU IHRER HERSTELLUNG
PHARMACEUTICAL COMPOSITIONS BASED ON DALFOPRISTIN AND QUINUPRISTIN AND PREPARATION

(30) Priorité: 16.12.1997 FR 9715911
(43) Date de publication de la demande: 27.09.2000
(73) Titulaire: Aventis Pharma S.A., 92160 Antony (FR)
(72) Inventeur: CONRATH, Guillaume, F-92290 Chatenay Malabry (FR); VACUS, Joel, F-75013 Paris (FR); BARKER, Nicholas, Paul, Southborough, MA 01772 (US)
(86) Numéro de dépôt international: FR9802740
(87) Numéro de publication internationale: WO99030728

(56) Documents cités:
- WO-A-98/22107
- CHANT C ET AL: "Quinupristin-dalfopristin (RP 59500): A new streptogramin antibiotic" ANNALS OF PHARMACOTHERAPY, 29 (10). 1995. 1022-1027., XP002079026
- BERNARD E ET AL: "PHARMACOKINETICS AND SUCTION BLISTER FLUID PENETRATION OF A SEMISYNTHETIC INJECTABLE STREPTOGRAMIN RP 59500 (RP 57669/RP 54476)" EUROPEAN JOURNAL OF CLINICAL MICROBIOLOGY & INFECTIOUS DISEASES, vol. 13, no. 9, septembre 1994, pages 768-771, XP002037989

## Description

La présente invention concerne des compositions pharmaceutiques injectables destinées à l'administration parentérale de quinupristine et de dalfopristine, sans entraîner d'effets secondaires d'intolérance au niveau de la zone d'injection.

Dans la demande de brevet EP 248 703 ont été décrits les dérivés de pristinamycine I de formule générale : 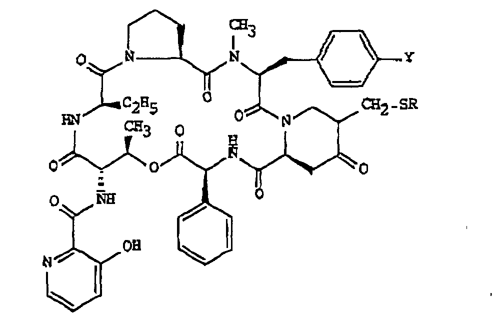 ainsi que leurs associations avec des dérivés de pristinamycine II de structure :

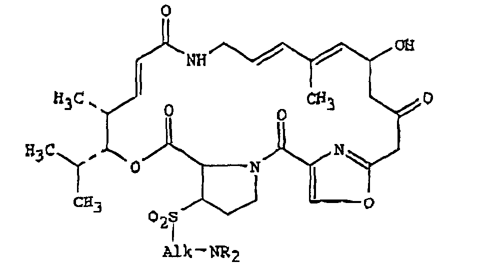

La quinupristine, dérivé de pristinamycine I, et la dalfopristine, dérivé de pristinamycine II, sont les composantes du Synercid®:

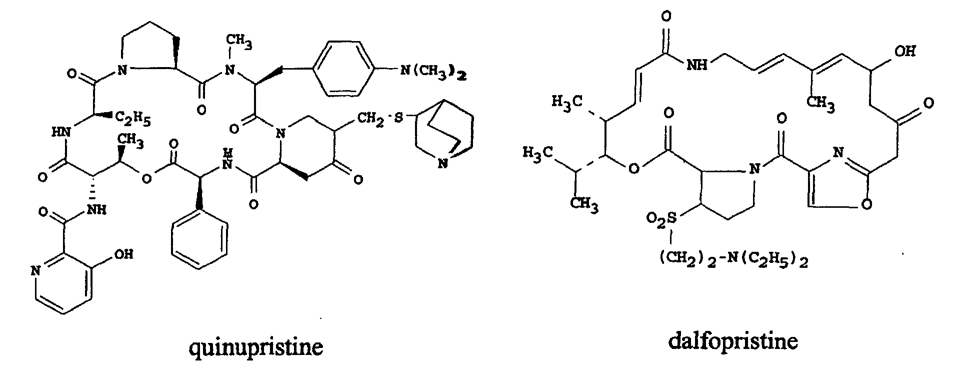

Le Synercid® (quinupristine / dalfopristine) est une combinaison 30/70, injectable, dont l'activité antibactérienne, notamment sur les germes résistants à la vancomycine, est citée dans de nombreuses publications [The Annals of Pharmacotherapy, 29, 1022-1026 (1995) ; Microbial Drug résistance, 1, 223-234 (1995)].

La solubilisation des composantes isolées quinupristine ou dalfopristine peut être obtenue à l'état de sel. La préparation de compositions pharmaceutiques stabilisées comprenant l'association quinupristine / dalfopristine s'est avérée très difficile et a pu être finalement réalisée par addition de quantités au moins stoechiométriques d'acide méthanesulfonique ou d'acide chlorhydrique, et à un pH compris dans l'intervalle [3,5;5]. Lesdites compositions contiennent éventuellement, en outre, un agent isotonisant et/ou d'autres adjuvants pharmaceutiquement acceptables.

Les tentatives de préparation de compositions pharmaceutiques comprenant la dalfopristine et la quinupristine sous forme d'autres sels s'est avérée être un échec du fait que l'une ou l'autre des molécules était instable soit en solution, soit sous forme lyophilisée.

L'injection (notamment l'injection par perfusion) des compositions pharmaceutiques comprenant l'association quinupristine / dalfopristine telles que décrites ci-dessus entraîne des effets d'intolérance veineuse localisée au voisinage du point d'injection qui se manifestent par des phénomènes inflammatoires, des phlébites, des réactions allergiques ou des formations d'oedèmes pouvant aller jusqu'à l'interruption totale du traitement. Une telle situation est extrêmement préoccupante du fait que le Synercid® (quinupristine / dalfopristine) s'avère être actuellement parmi les seuls traitements connus en clinique pour traiter les infections très graves occasionnées par des germes résistants à la vancomycine.

H. Yalkowsky et coll., PDA Journal of Pharmaceutical Science & Technology, 50(2), 123-128 (1996) a décrit l'étude et l'amélioration des phlébites occasionnées par l'administration d'un agent cardiovasculaire : le dexverapamil. Cependant cet agent thérapeutique possède une structure chimique très différente par comparaison aux structures de la famille des streptogramines et ne présente pas non plus de lien dans ses caractères physico-chimiques.

S.L. Gupta et coll., Journal of Pharmaceutical Science & Technology, 48(2), 86-91 (1994) a décrit les effets de différents systèmes en vue de pallier la douleur et l'irritation à l'injection provoquées par l'antihypertenseur Abbott 72517, parmi lesquels l'usage d'un tampon. Les formulations pharmaceutiques incluant le tampon sont également décrites. Cependant il s'agit non seulement d'un produit isolé et non de 2 molécules associées ayant chacune leur particularité, mais de plus il ne s'agit pas d'une structure chimiquement voisine permettant une transposition avec quelque chances de succès. De plus il n'a pas été possible de préparer des compositions pharmaceutiques stables de l'association quinupristine/dalfopristine avec l'acide citrique ou l'acide acétique.

Il a maintenant été trouvé et c'est ce qui fait l'objet de l'invention, que l'usage d'un additif, associé à l'injection de la composition pharmaceutique comprenant l'association quinupristine / dalfopristine, pouvait diminuer, voire supprimer complètement les effets secondaires localisés entraînés par cette association de principes actifs ; et ceci de manière surprenante, compte tenu de la nature très différente de chacune de molécules associées et malgré les difficultés liées à l'instabilité de certains sels de l'une ou l'autre de ces molécules (apparition de nombreuses impuretés de dégradation) et aussi à la mauvaise solubilité et à l'instabilité de ces principes actifs à certains pH.
Ainsi, ledit additif joue un rôle protecteur vis-à-vis des effets d'intolérance veineuse entraînés par l'injection de l'association quinupristine / dalfopristine

Les formulations de l'association quinupristine / dalfopristine sont présentées à l'état liquide, lyophilisé ou congelé.
Les solutions lyophilisées peuvent être reprises, au moment de l'emploi, par de l'eau pour préparations injectables (eau ppi) ou par tout milieu injectable compatible, notamment par des milieux tels que des solutions glucosées (solution aqueuse de glucose à 5 % par exemple), ou à titre non limitatif, par des solutions de dextrane, de polyvinylpyrrolidone ou de polysorbate 80. Selon un mode préféré la remise en solution est effectuée en passant par l'intermédiaire d'une solution concentrée (de 50 à 250 mg/ml, de préférence environ 100 mg/ml) appelée ci-après « concentrat » ; cette solution est diluée au moment de l'emploi dans un milieu injectable tel que décrit ci-dessus pour une administration par perfusion ; il est également possible de remettre en solution le lyophilisat dans de l'eau ppi puis de diluer le concentrat ainsi obtenu dans le milieu injectable souhaité.
Les formulations congelées peuvent être congelées à partir de solutions initialement préparées (5 à 250 mg/ml) ou à partir de solutions diluées (pour la préparation de poches congelées par exemple). Elles sont décongelées au moment de l'emploi, puis le cas échéant diluées.
Les solutions présentées à l'état liquide contiennent 5 à 250 mg/ml de principe actif. Elles sont diluées au moment de l'emploi à des concentrations comprises entre 0,5 et 10 mg/ml.

On entend ci-dessus par additif une solution tampon choisie parmi toute solution aqueuse pharmaceutiquement acceptable tamponnée à pH acide, capable de fixer le pH du milieu à une valeur inférieure au pH du plasma sanguin, et notamment à des valeurs où la stabilité de l'association quinupristine / dalfopristine n'est pas affectée, c'est-à-dire à des valeurs qui n'entraînent pas de dégradation immédiate ou rapide de l'un et/ou l'autre des principes actifs. De préférence on entend par additif toute solution pharmaceutiquement acceptable tamponnée à pH compris entre 3 et 6.
A titre préférentiel peut être citée toute solution pharmaceutiquement acceptable formée par un système acide/base dont l'un au moins des constituants est un acide faible ou une base faible, pharmaceutiquement acceptable, dont le pKa est compris dans l'intervalle [3 ; 6], et dont le pH résultant du système est voisin ou inférieur au pKa ci-dessus.
A titre encore plus préférentiel, le système peut être constitué d'un ou plusieurs acides faibles organiques ou minéraux pharmaceutiquement acceptables dont le pKa est compris dans l'intervalle [3 ; 6], associés à leur base conjuguée, à une base forte
ou à une base faible, ou bien le système peut être constitué d'un ou plusieurs acides forts organiques ou minéraux pharmaceutiquement acceptables, associés à au moins une base faible appartenant à un couple acide/base dont le pKa est compris dans l'intervalle [3 ; 6].

A titre d'exemple, peuvent entrer dans la composition du système, les acides donnés ci-après (ou leur base conjuguée) : acide citrique, acide acétique, acide lactique. acides aminés, acide malique, acide ascorbique, acide glutamique, acide benzoïque. histidine, acide glutarique, acide propionique, acide succinique, acide formique, acide maléique, acide aspartique, acide malonique, acide gluconique, acide glucoheptonique, acide phosphorique. Ces acides peuvent être associés à leur base conjuguée, à la base conjuguée d'un autre acide faible ou à de la soude ; les bases conjuguées des acides mentionnés ci-dessus peuvent aussi être le cas échéant associées à l'acide méthanesulfonique, l'acide chlorhydrique, l'acide phosphorique ou l'acide sulfurique.
Et parmi ces exemples, donnés à titre non limitatif, tout particulièrement intéressants sont les acides citrique, acétique, lactique, acides aminés et/ou leur base conjuguée.

Selon l'invention les formulations de l'association quinupristine / dalfopristine éventuellement reconstituées à l'état de solution concentrée (concentrat) ou diluées, peuvent être associées à une solution tampon au moment de l'injection. L'association peut être effectuée indifféremment entre le concentrat et la solution tampon (le cas échéant diluée préalablement), avant l'introduction dans la poche de perfusion. L'association peut être aussi effectuée directement dans la poche de perfusion, dans laquelle la solution tampon aura été préalablement introduite pour former tout d'abord une solution tampon diluée, puis par introduction du concentrat. Selon un autre alternative, la solution tampon peut également être introduite dans la poche contenant déjà la formulation du principe actif. L'association peut être aussi effectuée au moyen de 2 poches de perfusion contenant l'une le principe actif dans le milieu injectable et l'autre la solution tampon dissoute elle aussi dans le milieu injectable, les 2 poches étant reliées ensemble par un cathéter en Y.
Selon encore une autre alternative, le lyophilisat peut être remis en solution directement par une solution tampon diluée, puis le mélange dilué à nouveau dans tout milieu injectable compatible et pharmaceutiquement acceptable, par exemple directement dans la poche de perfusion, ou bien encore le lyophilisât peut être remis en solution par dilution directe dans un milieu injectable compatible contenant déjà la solution tampon (par exemple directement dans une poche de perfusion contenant la solution tampon dissoute dans le milieu injectable).
Dans le cas de l'emploi de poches congelées contenant déjà la solution diluée de principe actif, l'association sera formée par introduction de la solution tampon directement dans la poche.

Les doses de principe actif (association quinupristine + dalfopristine) administrées aux patients sont habituellement comprises entre 5 et 15 mg/kg et de préférence 5 à 7,5 mg/kg. Il conviendra d'associer des concentrations de solution tampon convenables pour exercer l'effet tampon et dans une limite et dans un volume tels que le seuil de tolérance du point de vue thérapeutique ne soit pas dépassés. Selon un aspect préféré de l'invention, les concentrations molaires en acide+base peuvent varier de 0,005 à à 2 mol/l dans un volume de 1 à 500 ml, le pH étant fixé entre 3 et 6 et plus spécialement entre 3,5 et 5. De préférence le pH est fixé entre 4 et 5 et plus particulièrement à 4. Dans certains cas il peut être éventuellement nécessaire de diluer ces solutions, de manière à administrer au patient une dose comprise entre 1 et 15 mmol.

Les solutions associées de principe actif et de solution tampon présentent une stabilité suffisante pour une administration extemporanée par perfusion. Notamment elles sont stables, sans dégradation notable pendant une durée de 6 heures à une température de 20°C ou pendant une durée de 24 heures à 4°C. Plus particulièrement, on entend par solution stable, une solution ne présentant pas d'apparition d'opalescence ou de particules (estimation visuelle ou mesure de la densité optique) après au moins 6 heures entre 25 et 30°C.

Les compositions pharmaceutiques stabilisées comprenant l'association quinupristine / dalfopristine sont préparées par mise en solution simultanée ou successive de la quinupristine, la dalfopristine, l'acide méthanesulfonique ou l'acide chlorhydrique dans de l'eau, puis ajustement du pH dans l'intervalle [3,5 ; 5] et/ou addition d'un agent isotonisant et/ou addition d'autres adjuvants pharmaceutiquement acceptables et le cas échéant lyophilisation et/ou congélation.
Lesdites compositions sont plus particulièrement préparées par dissolution de la composante quinupristine puis de la composante dalfopristine dans de l'eau acidifiée par l'acide méthanesulfonique ou l'acide chlorhydrique, suivie le cas échéant de l'ajustement du pH dans l'intervalle [3,5 ; 5] et/ou de l'addition d'un agent isotonisant et/ou d'autres adjuvants pharmaceutiquement acceptables. Elles sont le cas échéant lyophilisées et/ou congelées. La préparation et la répartition de la solution sont effectuées généralement de 0°C à la température ambiante, de préférence à basse température, cette température étant fonction de la durée de la préparation et du pH. On opère de préférence à une température inférieure à 10°C. Ces compositions pharmaceutiques stabilisées sont éventuellement stérilisées, notamment par filtration stérilisante.

Lorsque les compositions pharmaceutiques stabilisées contiennent un adjuvant pharmaceutiquement acceptable, ce dernier est choisi parmi les cosolvants, des stabilisants, des agents cryoprotecteurs, des agents dessicants, des charges et des agents isotonisants. A titre non limitatif les cosolvants et les agents solubilisants sont choisis parmi les polyéthylèneglycols (polyéthylèneglycols 300 et 400), le propylèneglycol, l'éthanol et les agents tensioactifs comme par exemple le polysorbate 80 ou les dérivés polyoxyéthylénés (crémophors) ; les charges et les agents cryoprotecteurs sont notamment choisis parmi les sucres simples (par exemple le glucose, le mannitol, le fructose, le sorbitol), les disaccharides (par exemple le saccharose, le lactose, le tréhalose, le maltose) ou les polymères hydrosolubles (par exemple les dextrans, la carboxyméthylcellulose, la polyvinylpyrrolidone ou la gélatine) ; les agents stabilisants sont notamment choisis parmi les agents anti-oxydants... ; les agents isotonisants sont notamment choisis parmi le glucose, le chlorure de sodium, le glycérol, le sorbitol, le mannitol, le fructose, ou les dextrans 40 et 70.

Selon l'invention, les solutions tampon peuvent être préparées selon les méthodes connues, habituellement utilisées. Notamment par addition de soude sur la quantité prédéterminée d'acide, jusqu'au pH désiré compris entre 3,5 et 5, puis addition d'eau ppi jusqu'au volume souhaité. Ces solutions peuvent en outre contenir un/des adjuvants compatibles et pharmaceutiquement acceptables, comme par exemple des agents tensioactifs.
Parmi les agents tensioactifs pouvant être utilisés, on peut citer à titre non limitatif les dérivés d'huile de ricin polyoxyéthylénée (cremophores par exemple), les esters de sorbitan polyhydroxyéthylés (comme les polysorbates : polysorbate 80 par exemple) ou les lécithines.
Lorsque l'on utilise de tels adjuvants dans la solution tampon, ces agents sont introduits de manière qu'ils constituent, dans l'association finale comprenant la solution de principe actif et la solution tampon, une quantité totale 1 à 25 mg par mg de l'association dalfopristine / quinupristine.

Il est entendu que les kits de présentation de la formulation de l'association quinupristine/dalfopristine et de l'additif entrent également dans le cadre de la présente invention. Toute forme de kits de présentation peuvent convenir, notamment, à titre d'exemple des présentations sous forme de double flaconnage, des présentations sous forme de poche de perfusion contenant l'additif et de flacon(s) contenant le lyophilisât, des présentations sous forme de poche de perfusion contenant le principe actif et d'un flacon ou d'une ampoule contenant l'additif, des présentations dans lesquelles intervient un ou plusieurs flacons comprenant le lyophilisat et un flacon ou une ampoule de l'additif. Des dispositifs du type seringue à double compartiment, peuvent aussi s'avérer particulièrement adaptés.

Il est entendu que la présente invention peut s'appliquer également aux autres dérivés solubles de la pristinamycine, particulièrement aux dérivés à chaîne aminée. Par exemple elle peut aussi s'appliquer à des dérivés tels que décrits dans les brevets européens EP 133097, EP 135410, EP 191662, EP 248703.

Les exemples suivants donnés à titre non limitatif montrent comment l'invention peut être mise en pratique.

### Essai 1

Une solution tampon est préparée à partir de 2,55 g d'acide citrique monohydraté et 2,59 g de citrate trisodique dihydraté, complétés à 100 ml par de l'eau ppi. La solution ainsi préparée a un pH de 4,0 et une molarité en acide citrique d'environ 210 mmol/l.

Un flacon de Synercid® (association 70/30 dalfopristine / quinupristine) est reconstitué à partir de 550 mg de lyophilisât de l'association 70/30 dalfopristine / quinupristine, par addition de 5 ml d'eau ppi.
1ml de cette solution est introduit dans 24 ml de la solution tampon préparée précédemment. Après homogénéisation manuelle, la solution est soumise à une filtration stérilisante sur filtre de porosité 0,22 µm (filtre Millipore Millex SLGV). La stabilité chimique de la solution est acceptable.

### Description du test - modèle de la queue de rat :

Le Synercid® injecté par voie intradermale dans la veine de la queue de rats observés pendant 10 jours, provoque à une concentration de 4 mg/ml, une rougeur puis une nécrose. La rougeur apparaît au dessus du point d'injection pendant 2 à 3 jours, elle est suivie d'une nécrose aux jours 4 à 6.
On évalue l'hématome, l'érythème, l'oedème et la nécrose sur une échelle de 1 à 4 [1 = minimum ; 2 = léger ; 3 = modéré ; 4 = sévère]. La sévérité globale est appréciée au regard du score le plus élevé observé parmi les 4 observations. L'appréciation de la tolérance s'exprime par la moyenne des scores obtenus pour tous les animaux ayant reçu un traitememt identique.

### Mise en oeuvre du test et résultats :

La solution constituée comme décrit ci-dessus est administrée chez le rat dans le modèle décrit. Les résultats de l'étude montrent que toutes les réactions secondaires (rougeurs, nécroses et perte de la queue) ont été supprimées par rapport aux rats témoins ayant reçu la même dose de Synercid® dans une solution de glucose à 5 %.

| Traitement | Jour 1 | Jour 2 | Jour 3 | Jour 4 | Jour 5 | Jour 6 | Jour 7 | Jour 8 |
|---|---|---|---|---|---|---|---|---|
| Synercid®/Glucose à 5% | 0,0 (6) | 2,0 (5) | 2,5 (4) | 3,7 (4) | 3,7 (4) | 3,7 (4) | 3,7 (4) | 3,7 (3) |
| Synercid®/tampon citrate 210 mmol/l | 0,0 (6) | 0,0 (5) | 0,0 (5) | 2,0 (5) | 2,0 (5) | 0,0 (5) | 0,0 (5) | 0,0 (5) |
| () nombre d'animaux survivants | | | | | | | | |

Le lyophilisât de dalfopristine / quinupristine peut être préparé de la manière suivante :

On prépare un litre d'une solution à 125 mg/ml de quinupristine/ dalfopristine (30/70), salifiée par de l'acide méthanesulfonique (≈ 16,7 mg/ml) à un pH de 4,75 en introduisant 810 g d'eau pour préparation injectable dans une cuve de dissolution équipée d'une station de réfrigération. La solution est réfrigérée à une température comprise entre 0 et 6°C tout au long de la fabrication. On ajoute 16,4 g d'acide méthanesulfonique, puis on introduit successivement 37,5 g de quinupristine qui sont dissous par agitation mécanique et 87,5 g de dalfopristine qui sont également dissous par agitation mécanique. Le pH de la solution est ajusté à 4,75 par une solution IN d'acide méthanesulfonique. La solution est complétée à 1 litre (1030 g) avec de l'eau pour préparation injectable.

Cette solution est stérilisée par filtration stérilisante (filtre 0,22 µm) et répartie dans des flacons [500 mg de quinupristine/ dalfopristine (30/70) par flacon] puis lyophilisée. [congélation : température -30°C à -50°C; vitesse de congélation environ 0,5°/min. Sublimation : pression 0,5 mbar. Dessiccation secondaire : pression (≈ 30 µbars) température 40°C].

### Essais 2 à 5

En opérant comme pour l'essai 1 et en diluant la solution tampon par une solution de glucose à 5 %, on obtient les résultats suivants :

| Traitement | Jour 1 | Jour 2 | Jour 3 | Jour 4 | Jour 5 | Jour 6 | Jour 7 | Jour 8 |
|---|---|---|---|---|---|---|---|---|
| Synercid®/Glucose à 5% | 0,5 (6) | 1,8 (5) | 2,4 (5) | 2,8 (5) | 2,6 (5) | 2,6 (5) | 2,6 (5) | 2,4 (5) |
| Synercid®/tampon citrate 50 mmol/l | 0,2 (6) | 0,5 (6) | 0,3 (6) | 0,5 (6) | 0,3 (6) | 2,0 (6) | 0,0 (6) | 0,0 (6) |
| Synercid®/tampon citrate 25 mmol/l | 0,2 (6) | 0,8 (6) | 0,5 (6) | 0,3 (6) | 0,3 (6) | 0,2 (6) | 0,2 (6) | 0,0 (6) |
| Synercid®/tampon citrate 12,5 mmol/l | 0,5 (6) | 0,8 (6) | 0,5 (6) | 0,5 (6) | 0,3 (6) | 0,2 (6) | 0,2 (6) | 0,2 (6) |
| Synercid®/tampon citrate 6,25 mmol/l | 0,2 (6) | 0,2 (6) | 0,3 (6) | 0,3 (6) | 0,3 (6) | 0,2 (6) | 0,0 (6) | 0,0 (6) |
| () nombre d'animaux survivants | | | | | | | | |

Les résultats de l'étude montrent que toutes les réactions secondaires (rougeurs, nécroses et perte de la queue) ont été supprimées ou fortement diminuées par rapport aux rats témoins ayant reçu la même dose de Synercid® dans une solution de glucose à 5 %.

### Essai 6

Un essai identique à l'essai 1 décrit ci-dessus est mis en oeuvre en préparant une solution tampon contenant 0,35 ml d'acide acétique à 99%, 1,92 g d'acétate de sodium trihydraté, 0,19 g de chlorure de sodium, complétés à 100 ml par de l'eau ppi. La solution ainsi préparée a un pH de 5,02.

Après filtration stérilisante de la solution comme décrit ci-dessus, la stabilité chimique de la solution est acceptable.

### Essai 7 : étude clinique

La dose de 7,5 mg/kg de Synercid® (association 70/30 dalfopristine / quinupristine) reprise pendant 1 heure dans un volume de 250 ml de solution aqueuse de glucose à 5% correspond à la dose unitaire utilisée en clinique par voie i.v..

Une solution de tampon citrate acide à 20 mM/l est préparée (5 mM dans une solution de 250 ml contenant 1051 mg d'acide citrique monohydraté).

Un traitement de 48 heures est habituellement suffisant pour voir apparaître les réactions d'intolérance veineuse. 8 sujets par groupe sont sélectionnés. Les critères d'interruption du traitement sont définis préalablement, basés sur une douleur importante, une induration de la veine ou la décision du patient ou de l'investigateur d'interrompre le traitement.

Après 2 jours de traitement des douleurs ont été observées ainsi que de érythèmes au niveau du site d'injection, mais aucun phénomène de thrombose veineuse n'est apparu et aucune interruption de traitement n'a dû être décidée. La conclusion de l'essai, en accord avec le protocole défini, est une nette amélioration de la tolérance générale à cette concentration de tampon citrate.

## Revendications

1. Compositions injectables contenant l'association dalfopristine / quinupristine **caractérisées en ce qu'**elles comprennent une solution aqueuse contenant l'association dalfopristine / quinupristine et un additif choisi parmi toute solution aqueuse pharmaceutiquement acceptable tamponnée à pH acide, capable de fixer le pH du milieu à une valeur inférieure au pH du plasma sanguin, et à des valeurs où la stabilité de l'association dalfopristine / quinupristine n'est pas affectée.

2. Compositions injectables selon la revendication 1, **caractérisées en ce que** l'additif est choisi parmi toute solution pharmaceutiquement acceptable tamponnée à pH compris entre 3 et 6.

3. Compositions injectables selon la revendication 1 ou 2, **caractérisées en ce que** l'additif est choisi parmi toute solution pharmaceutiquement acceptable, formée par un système acide/base dont l'un au moins des constituants est un acide faible ou une base faible pharmaceutiquement acceptable, dont le pKa est compris dans l'intervalle [3 ; 6], et dont le pH résultant du système est voisin ou inférieur audit pKa.

4. Compositions injectables selon l'une des revendications 1 à 3, **caractérisées en ce que** le pH de la solution tampon est fixé entre 3,5 et 5.

5. Compositions injectables selon la revendication 4, **caractérisées en ce que** le pH de la solution tampon est fixé entre 4 et 5.

6. Compositions injectables selon l'une des revendications 4 ou 5, **caractérisées en ce que** le pH de la solution tampon est fixé à environ 4.

7. Compositions injectables selon l'une des revendications 1 à 6, **caractérisées en ce que** l'additif est choisi parmi toute solution pharmaceutiquement acceptable, formée par un système acide/base constitué d'un ou plusieurs acides faibles organiques ou minéraux pharmaceutiquement acceptables dont le pKa est compris dans l'intervalle [3 ; 6], associés à leur base conjuguée, à une base forte ou à une base faible, ou formée par un système acide/base constitué d'un ou plusieurs acides forts organiques ou minéraux pharmaceutiquement acceptables, associés à au moins une base faible appartenant à un couple acide/base dont le pKa est compris dans l'intervalle [3 ; 6].

8. Compositions injectables selon la revendication 7, **caractérisées en ce que** le système comprend des acides, ou leur base conjuguée, choisis parmi l'acide citrique, l'acide acétique, l'acide lactique, les acides aminés, l'acide malique, l'acide ascorbique, l'acide glutamique, l'acide benzoïque, l'histidine, l'acide glutarique, l'acide propionique, l'acide succinique, l'acide formique, l'acide maléique, l'acide aspartique, l'acide malonique, l'acide gluconique, l'acide glucoheptonique, l'acide phosphorique, associés à leur base conjuguée, à la base conjuguée d'un autre acide faible ou à de la soude lorsqu'il s'agit des acides ou associés à l'acide méthanesulfonique, l'acide chlorhydrique, l'acide phosphorique ou l'acide sulfurique lorsqu'il s'agit de la base conjuguée des acides précédemment énumérés.

9. Compositions injectables selon la revendication 8, **caractérisées en ce que** le système comprend les acides citrique, acétique, lactique, les acides aminés et/ou leur base conjuguée.

10. Compositions injectables selon l'une des revendications 1 à 8, **caractérisées en ce que** la solution tampon comprend en outre un agent tensioactif.

11. Compositions injectables selon la revendication 10, **caractérisées en ce que** l'agent tensioactif est choisi parmi les dérivés d'huile de ricin polyoxyéthylénée, les esters de sorbitan polyhydroxyéthylés ou les lécithines.

12. Compositions injectables selon la revendication 10, **caractérisées en ce que** l'agent tensioactif est le polysorbate 80.

13. Compositions injectables à double compartiment destinées à la préparation d'une solution pour perfusion contenant l'association dalfopristine / quinupristine **caractérisées en ce qu'**elles comprennent d'une part une solution aqueuse contenant l'association dalfopristine / quinupristine et d'autre part un additif choisi parmi toute solution aqueuse pharmaceutiquement acceptable tamponnée à pH acide, capable de fixer le pH du milieu à une valeur inférieure au pH du plasma sanguin, et à des valeurs où la stabilité de l'association dalfopristine / quinupristine n'est pas affectée.

14. Compositions injectables selon l'une des revendications 1 à 13, **caractérisées en ce que** l'association dalfopristine / quinupristine est constituée de la dalfopristine et de la quinupristine additionées de quantités au moins stoechiométriques d'acide méthanesulfonique ou d'acide chlorhydrique, et à un pH compris dans l'intervalle [3,5; 5].

15. Utilisation d'une solution tampon choisie parmi toute solution aqueuse pharmaceutiquement acceptable tamponnée à pH acide, capable de fixer le pH du milieu à une valeur inférieure au pH du plasma sanguin, et à des valeurs où la stabilité de l'association dalfopristine / quinupristine n'est pas affectée, pour la préparation d'un médicament destiné à protéger vis à vis des effets d'intolérance veineuse entraînés par l'injection de l'association quinupristine / dalfopristine.

16. Utilisation selon la revendication 15, d'une solution tampon choisie parmi toute solution pharmaceutiquement acceptable tamponnée à pH compris entre 3 et 6, pour la préparation d'un médicament destiné à protéger vis à vis des effets d'intolérance veineuse entraînés par l'injection de l'association quinupristine / dalfopristine.

17. Utilisation selon l'une des revendications 15 ou 16, d'une solution tampon choisie parmi les solutions formées par un système acide/base dont l'un au moins des constituants est un acide faible ou une base faible, pharmaceutiquement acceptable, dont le pKa est compris dans l'intervalle [3 ; 6], et dont le pH résultant du système est voisin ou inférieur audit pKa, pour la préparation d'un médicament destiné à protéger vis à vis des effets d'intolérance veineuse entraînés par l'injection de l'association quinupristine / dalfopristine.

18. Utilisation selon l'une des revendications 15, 16 ou 17, d'une solution tampon choisie parmi les solutions formées par un système acide/base constitué d'un ou plusieurs acides faibles organiques ou minéraux pharmaceutiquement acceptables dont le pKa est compris dans l'intervalle [3 ; 6], associés à leur base conjuguée, à une base forte ou à une base faible, ou formées par un système acide/base constitué d'un ou plusieurs acides forts organiques ou minéraux pharmaceutiquement acceptables, associés à au moins une base faible appartenant à un couple acide/base dont le pKa est compris dans l'intervalle [3 ; 6], pour la préparation d'un médicament destiné à protéger vis à vis des effets d'intolérance veineuse entraînés par l'injection de l'association quinupristine / dalfopristine.

19. Utilisation selon l'une des revendications 15, 16, 17 ou 18, d'une solution tampon formée par un système acide/base constitué d'un acide, ou sa base conjuguée, choisi parmi l'acide citrique, l'acide acétique, l'acide lactique, les acides aminés, l'acide malique, l'acide ascorbique, l'acide glutamique, l'acide benzoïque, l'histidine, l'acide glutarique, l'acide propionique, l'acide succinique, l'acide formique, l'acide maléique, l'acide aspartique, l'acide malonique, l'acide gluconique, l'acide glucoheptonique, l'acide phosphorique, associés à sa base conjuguée, à la base conjuguée d'un autre acide faible ou à de la soude lorsqu'il s'agit des acides ou associés à l'acide méthanesulfonique, l'acide chlorhydrique, l'acide phosphorique ou l'acide sulfurique lorsqu'il s'agit de la base conjuguée des acides précédemment énumérés, pour la préparation d'un médicament destiné à protéger vis à vis des effets d'intolérance veineuse entraînés par l'injection de l'association quinupristine / dalfopristine.

20. Kit de présentation de la formulation de l'association quinupristine / dalfopristine et d'un additif choisi parmi toute solution aqueuse pharmaceutiquement acceptable tamponnée à pH acide, capable de fixer le pH du milieu à une valeur inférieure au pH du plasma sanguin, et à des valeurs où la stabilité de l'association dalfopristine / quinupristine n'est pas affectée.

21. Kit de présentation selon la revendication 20 **caractérisé en ce que** l'association dalfopristine / quinupristine est constituée de la dalfopristine et de la quinupristine additionées de quantités au moins stoechiométriques d'acide méthanesulfonique ou d'acide chlorhydrique, et à un pH compris dans l'intervalle [3,5 ; 5].

22. Kit de présentation selon la revendication 20 ou 21 **caractérisé en ce qu'**il s'agit d'un double flaconnage, d'une poche de perfusion contenant l'additif et de flacon(s) contenant le lyophilisat, d'une poche de perfusion contenant le principe actif et d'un flacon ou d'une ampoule contenant l'additif, d'un ou plusieurs flacons comprenant le lyophilisat et un flacon ou une ampoule contenant l'additif, d'une seringue à double compartiment.

## Patentansprüche

1. Injizierbare Zusammensetzungen, enthaltend die Assoziation Dalfopristin/Quinupristin, **dadurch gekennzeichnet, daß** sie eine wäßrige Lösung umfassen, die die Assoziation Dalfopristin/Quinupristin und einen Zusatzstoff enthält, ausgewählt unter jeder pharmazeutisch akzeptablen wäßrigen und auf saurem pH-Wert gepufferten Lösung, die fähig ist, den pH des Milieus auf einem Wert von unterhalb dem pH des Blutplasmas und auf Werte zu fixieren, wo die Stabilität der Assoziation Dalfopristin/Quinupristin nicht beeinträchtigt wird.

2. Injizierbare Zusammensetzungen nach Anspruch 1, **dadurch gekennzeichnet, daß** der Zusatzstoff unter jeder pharmazeutisch akzeptablen Lösung ausgewählt wird, die auf einen pH zwischen 3 und 6 gepuffert ist.

3. Injizierbare Zusammensetzungen nach Anspruch 1 oder 2, **dadurch gekennzeichnet, daß** der Zusatzstoff unter jeder pharmazeutisch akzeptablen Lösung ausgewählt wird, die aus einem System Säure/Base gebildet ist und bei der mindestens einer der Bestandteile eine pharmazeutisch akzeptable schwache Säure oder eine schwache Base ist, deren pKa sich im Intervall von [3;6] befindet und deren resultierender pH des Systems in der Nähe oder unterhalb des genannten pKa liegt.

4. Injizierbare Zusammensetzungen nach einem der Ansprüche 1 bis 3, **dadurch gekennzeichnet, daß** der pH der Pufferlösung zwischen 3,5 und 5 fixiert ist.

5. Injizierbare Zusammensetzungen nach Anspruch 4, **dadurch gekennzeichnet, daß** der pH der Pufferlösung zwischen 4 und 5 fixiert ist.

6. Injizierbare Zusammensetzungen nach einem der Ansprüche 4 oder 5, **dadurch gekennzeichnet, daß** der pH der Pufferlösung auf etwa 4 fixiert ist.

7. Injizierbare Zusammensetzungen nach einem der Ansprüche 1 bis 6, **dadurch gekennzeichnet, daß** der Zusatzstoff unter jeder pharmazeutisch akzeptablen Lösung ausgewählt wird, die aus einem System Säure/Base gebildet ist, das aus einer oder mehreren pharmazeutisch akzeptablen schwachen organischen Säuren oder Mineralsäuren besteht, deren pKa sich im Intervall von [3;6] befindet, assoziiert mit ihrer konjugierten Base, mit einer starken Base oder mit einer schwachen Base, oder gebildet durch ein System Säure/Base, bestehend aus einer oder mehreren pharmazeutisch akzeptablen starken organischen Säuren oder Mineralsäuren, assoziiert mit mindestens einer schwachen Base, die aus einem Paar Säure/Base stammt, dessen pKa im Intervall von [3;6] liegt.

8. Injizierbare Zusammensetzungen nach Anspruch 7, **dadurch gekennzeichnet, daß** das System Säuren oder ihre konjugierte Base umfaßt, ausgewählt unter Citronensäure, Essigsäure, Milchsäure, den Aminosäuren, Äpfelsäure, Ascorbinsäure, Glutaminsäure, Benzoesäure, Histidin, Glutarsäure, Propionsäure, Bernsteinsäure, Ameisensäure, Maleinsäure, Asparaginsäure, Malonsäure, Gluconsäure, Glucoheptonsäure, Phosphorsäure, assoziiert mit ihrer konjugierten Base, mit der konjugierten Base einer anderen schwachen Säure oder mit Natriumhydroxid, wenn es sich um Säuren handelt, oder assoziiert mit Methansulfonsäure, Chlorwasserstoffsäure, Phosphorsäure oder Schwefelsäure, wenn es sich um die konjugierte Base der vorstehend aufgezählten Säuren handelt.

9. Injizierbare Zusammensetzungen nach Anspruch 8, **dadurch gekennzeichnet, daß** das System Citronensäure, Essigsäure, Milchsäure, die Aminosäuren und/oder ihre konjugierte Base umfaßt.

10. Injizierbare Zusammensetzungen nach einem der Ansprüche 1 bis 8, **dadurch gekennzeichnet, daß** die Pufferlösung außerdem ein oberflächenaktives Mittel umfaßt.

11. Injizierbare Zusammensetzungen nach Anspruch 10, **dadurch gekennzeichnet, daß** das oberflächenaktive Mittel unter den Derivaten von polyoxyethyleniertem Ricinusöl, den Estern von polyhydroxyethyliertem Sorbitan oder den Lecithinen ausgewählt wird.

12. Injizierbare Zusammensetzungen nach Anspruch 10, **dadurch gekennzeichnet, daß** das oberflächenaktive Mittel Polysorbat 80 ist.

13. Injizierbare Zusammensetzungen mit zwei Fächern, bestimmt zur Herstellung einer Lösung für die Perfusion, enthaltend die Assoziation Dalfopristin/Quinupristin, **dadurch gekennzeichnet, daß** sie einerseits eine wäßrige Lösung, die die Assoziation Dalfopristin/Quinupristin enthält, und andererseits einen Zusatzstoff umfassen, ausgewählt unter jeder pharmazeutisch akzeptablen wäßrigen und auf saurem pH-Wert gepufferten Lösung, die fähig ist, den pH des Milieus auf einem Wert von unterhalb dem pH des Blutplasmas und auf Werte zu fixieren, wo die Stabilität der Assoziation Dalfopristin/Quinupristin nicht beeinträchtigt wird.

14. Injizierbare Zusammensetzungen nach einem der Ansprüche 1 bis 13, **dadurch gekennzeichnet, daß** die Assoziation Dalfopristin/Quinupristin aus Dalfopristin und aus Quinupristin, zugesetzt zu mindestens stöchiometrischen Mengen von Methansulfonsäure oder Chlorwasserstoffsäure bei einem pH-Wert im Intervall [3,5;5], gebildet wird.

15. Verwendung einer Pufferlösung, ausgewählt unter jeder pharmazeutisch akzeptablen wäßrigen und auf saurem pH-Wert gepufferten Lösung, die fähig ist, den pH des Milieus auf einem Wert von unterhalb dem pH des Blutplasmas und auf Werte zu fixieren, wo die Stabilität der Assoziation Dalfopristin/Quinupristin nicht beeinträchtigt wird, für die Herstellung eines Arzneimittels, das für den Schutz gegenüber Auswirkungen der venösen Intoleranz, verursacht durch die Injektion der Assoziation Dalfopristin/Quinupristin, vorgesehen ist.

16. Verwendung nach Anspruch 15 einer Pufferlösung, ausgewählt unter jeder pharmazeutisch akzeptablen und auf einem pH-Wert zwischen 3 und 6 gepufferten Lösung, für die Herstellung eines Arzneimittels, das für den Schutz gegenüber Auswirkungen der venösen Intoleranz, verursacht durch die Injektion der Assoziation Dalfopristin/Quinupristin, vorgesehen ist.

17. Verwendung nach einem der Ansprüche 15 oder 16 einer Pufferlösung, ausgewählt unter Lösungen, die aus einem System Säure/Base gebildet sind und bei denen mindestens einer der Bestandteile eine pharmazeutisch akzeptable schwache Säure oder eine schwache Base ist, deren pKa sich im Intervall von [3;6] befindet und deren resultierender pH des Systems in der Nähe oder unterhalb des genannten pKa liegt, für die Herstellung eines Arzneimittels, das für den Schutz gegenüber Auswirkungen der venösen Intoleranz, verursacht durch die Injektion der Assoziation Dalfopristin/Quinupristin, vorgesehen ist.

18. Verwendung nach einem der Ansprüche 15, 16 oder 17 einer Pufferlösung, ausgewählt unter Lösungen, die aus einem System Säure/Base gebildet sind, das aus einer oder mehreren pharmazeutisch akzeptablen schwachen organischen Säuren oder Mineralsäuren besteht, deren pKa sich im Intervall von [3;6] befindet, assoziiert mit ihrer konjugierten Base, mit einer starken Base oder mit.einer schwachen Base, oder gebildet durch ein System Säure/Base, bestehend aus einer oder mehreren pharmazeutisch akzeptablen starken organischen Säuren oder Mineralsäuren, assoziiert mit mindestens einer schwachen Base, die aus einem Paar Säure/Base stammt, dessen pKa im Intervall von [3;6] liegt, für die Herstellung eines Arzneimittels, das für den Schutz gegenüber Auswirkungen der venösen Intoleranz, verursacht durch die Injektion der Assoziation Dalfopristin/Quinupristin, vorgesehen ist.

19. Verwendung nach einem der Ansprüche 15, 16, 17 oder 18 einer Pufferlösung, gebildet durch ein System Säure/Base, das aus einer Säure oder ihrer konjugierten Base besteht, ausgewählt unter Citronensäure, Essigsäure, Milchsäure, den Aminosäuren, Äpfelsäure, Ascorbinsäure, Glutaminsäure, Benzoesäure, Histidin, Glutarsäure, Propionsäure, Bernsteinsäure, Ameisensäure, Maleinsäure, Asparaginsäure, Malonsäure, Gluconsäure, Glucoheptonsäure, Phosphorsäure, assoziiert mit ihrer konjugierten Base, mit der konjugierten Base einer anderen schwachen Säure oder mit Natriumhydroxid, wenn es sich um Säuren handelt, oder assoziiert mit Methansulfonsäure, Chlorwasserstoffsäure, Phosphorsäure oder Schwefelsäure, wenn es sich um die konjugierte Base der vorstehend aufgezählten Säuren handelt, für die Herstellung eines Arzneimittels, das für den Schutz gegenüber Auswirkungen der venösen Intoleranz, verursacht durch die Injektion der Assoziation Dalfopristin/Quinupristin, vorgesehen ist.

20. Darreichungs-Kit für die Formulierung der Assoziation Dalfopristin/Quinupristin und eines Zusatzstoffes, ausgewählt unter jeder pharmazeutisch akzeptablen wäßrigen und auf saurem pH-Wert gepufferten Lösung, die fähig ist, den pH des Milieus auf einem Wert von unterhalb dem pH des Blutplasmas und auf Werte zu fixieren, wo die Stabilität der Assoziation Dalfopristin/Quinupristin nicht beeinträchtigt wird.

21. Darreichungs-Kit nach Anspruch 20, **dadurch gekennzeichnet, daß** die Assoziation Dalfopristin/Quinupristin aus Dalfopristin und aus Quinupristin, zugesetzt zu mindestens stöchiometrischen Mengen von Methansulfonsäure oder Chlorwasserstoffsäure bei einem pH-Wert im Intervall [3,5;5], gebildet wird.

22. Darreichungs-Kit nach Anspruch 20 oder 21, **dadurch gekennzeichnet, daß** es sich um ein doppeltes Fläschchen, einen Perfusionsbeutel, der den Zusatzstoff und das (die) das Lyophilisat enthaltende(n) Fläschchen enthält, einen Perfusionsbeutel, der den Wirkstoff und ein Fläschchen oder eine Ampulle mit dem Zusatzstoff enthält, ein oder mehrere Fläschchen, die das Lyophilisat und ein Fläschchen oder eine Ampulle mit dem Zusatzstoff enthalten, oder eine Spritze mit zwei Fächern handelt.

## Claims

1. Injectable compositions containing the dalfopristine/quinupristine combination, **characterized in that** they comprise an aqueous solution containing the dalfopristine/quinupristine combination and an additive chosen from any pharmaceutically acceptable aqueous solution buffered to acidic pH, capable of fixing the pH of the medium at a value below the pH of the blood plasma, and at values at which the stability of the dalfopristine/quinupristine combination is not affected.

2. Injectable compositions according to Claim 1,
**characterized in that** the additive is chosen from any pharmaceutically acceptable solution buffered to a pH of between 3 and 6.

3. Injectable compositions according to either of Claims 1 and 2, **characterized in that** the additive is chosen from any pharmaceutically acceptable solution formed by an acid/base system in which at least one of the constituents is a pharmaceutically acceptable weak acid or weak base whose pKa is within the range [3; 6], and in which the resultant pH of the system is in the region of or below the said pKa.

4. Injectable compositions according to one of Claims 1 to 3, **characterized in that** the pH of the buffer solution is fixed between 3.5 and 5.

5. Injectable compositions according to Claim 4,
**characterized in that** the pH of the buffer solution is fixed between 4 and 5.

6. Injectable compositions according to either of Claims 4 and 5, **characterized in that** the pH of the buffer solution is fixed at about 4.

7. Injectable compositions according to one of Claims 1 to 6, **characterized in that** the additive is chosen from any pharmaceutically acceptable solution formed by an acid/base system consisting of one or more pharmaceutically acceptable weak organic or inorganic acids whose pKa is within the range [3; 6], combined with their conjugate base, with a strong base or with a weak base, or formed by an acid/base system consisting of one or more pharmaceutically acceptable strong organic or inorganic acids, combined with at least one weak base belonging to an acid/base couple whose pKa is within the range [3; 6].

8. Injectable compositions according to Claim 7, **characterized in that** the system comprises acids, or their conjugate base, chosen from citric acid, acetic acid, lactic acid, amino acids, malic acid, ascorbic acid, glutamic acid, benzoic acid, histidine, glutaric acid, propionic acid, succinic acid, formic acid, maleic acid, aspartic acid, malonic acid, gluconic acid, glucoheptonic acid, phosphoric acid, associated with their conjugate base, with the conjugate base of another weak acid or with sodium hydroxide when they are acids, or combined with methanesulphonic acid, hydrochloric acid, phosphoric acid or sulphuric acid when it is the conjugate base of the acids listed above.

9. Injectable compositions according to Claim 8, **characterized in that** the system comprises citric acid, acetic acid, lactic acid, amino acids and/or their conjugate base.

10. Injectable compositions according to one of Claims 1 to 8, **characterized in that** the buffer solution also comprises a surfactant.

11. Injectable compositions according to Claim 10, **characterized in that** the surfactant is chosen from polyoxyethylenated castor oil derivatives, polyhydroxyethylated sorbitan esters or lecithins.

12. Injectable compositions according to Claim 10, **characterized in that** the surfactant is polysorbate 80.

13. Injectable compositions with a twin-compartment intended for the preparation of a solution for infusion containing the dalfopristine/quinupristine combination, **characterized in that** they comprise, on the one hand, an aqueous solution containing the dalfopristine/quinupristine combination, and, on the other hand, an additive chosen from any pharmaceutically acceptable aqueous solution buffered to acidic pH, capable of fixing the pH of the medium at a value below the pH of the blood plasma, and at values at which the stability of the dalfopristine/quinupristine combination is not affected.

14. Injectable compositions according to one of Claims 1 to 13, **characterized in that** the dalfopristine/quinupristine combination consists of dalfopristine and quinupristine to which are added at least stoichiometric amounts of methanesulphonic acid or hydrochloric acid, and at a pH within the range [3.5; 5].

15. Use of a buffer solution chosen from any pharmaceutically acceptable aqueous solution buffered to acidic pH, capable of fixing the pH of the medium at a value below the pH of the blood plasma, and at values at which the stability of the dalfopristine/quinupristine combination is not affected, for the preparation of a medicinal product intended to protect against the venous intolerance effects entailed by the injection of the quinupristine/dalfopristine combination.

16. Use according to Claim 15, of a buffer solution chosen from any pharmaceutically acceptable solution buffered to a pH of between 3 and 6, for the preparation of a medicinal product intended to protect against the venous intolerance effects entailed by the injection of the quinupristine/dalfopristine combination.

17. Use according to either of Claims 15 and 16, of a buffer solution chosen from the solutions formed by an acid/base system in which at least one of the constituents is a pharmaceutically acceptable weak acid or weak base whose pKa is within the range [3; 6], and in which the resultant pH of the system is in the region of or below the said pKa, for the preparation of a medicinal product intended to protect against the venous intolerance effects entailed by the injection of the quinupristine/dalfopristine combination.

18. Use according to one of Claims 15, 16 and 17, of a buffer solution chosen from the solutions formed by an acid/base system consisting of one or more pharmaceutically acceptable weak organic or inorganic acids whose pKa is within the range [3; 6], combined with their conjugate base, with a strong base or with a weak base, or formed by an acid/base system consisting of one or more pharmaceutically acceptable strong organic or inorganic acids, combined with at least one. weak base belonging to an acid/base couple whose pKa is within the range [3; 6], for the preparation of a medicinal product intended to protect against the venous intolerance effects entailed by the injection of the quinupristine/dalfopristine combination.

19. Use according to one of Claims 15, 16, 17 and 18, of a buffer solution formed by an acid/base system consisting of an acid, or its conjugate base, chosen from citric acid, acetic acid, lactic acid, amino acids, malic acid, ascorbic acid, glutamic acid, benzoic acid, histidine, glutaric acid, propionic acid, succinic acid, formic acid, maleic acid, aspartic acid, malonic acid, gluconic acid, glucoheptonic acid, phosphoric acid, associated with its conjugate base, with the conjugate base of another weak acid or with sodium hydroxide when they are acids, or combined with methanesulphonic acid, hydrochloric acid, phosphoric acid or sulphuric acid when it is the conjugate base of the acids listed above, for the preparation of a medicinal product intended to protect against the venous intolerance effects entailed by the injection of the quinupristine/dalfopristine combination.

20. Presentation kit for the formulation of the quinupristine/dalfopristine combination and for an additive chosen from any pharmaceutically acceptable solution buffered to acidic pH, capable of fixing the pH of the medium at a value below the pH of the blood plasma, and at values at which the stability of the dalfopristine/quinupristine combination is not affected.

21. Presentation kit according to Claim 20, **characterized in that** the dalfopristine/quinupristine combination consists of dalfopristine and quinupristine to which are added at least stoichiometric amounts of methanesulphonic acid or hydrochloric acid, and at a pH within the range [3.5; 5].

22. Presentation kit according to Claim 20 or 21, **characterized in that** it is a twin-bottle, an infusion bag containing the additive and bottle(s) containing the lyophilizate, an infusion bag containing the active principle and a bottle or an ampoule containing the additive, one or more bottles comprising the lyophilizate and a bottle or an ampoule containing the additive, or a two-compartment syringe.
